# EUROPEAN PATENT APPLICATION

(11) **EP 2 119 466 A1**
(43) Date of publication of application: **18.11.2009**
(21) Application number: 08425327.7
(22) Date of filing: 12.05.2008
(51) Int. Cl.: A61M 16/16

(54) **Humidifier with safety elements**

(71) Applicant: Deas S.R.L., 48014 Castel Bolognese RA (IT)
(72) Inventor: Scardovi, Domenico, 48014 Castel Bolognese (Province of Ravenna) (IT)
(74) Representative: Modiano, Micaela Nadia

(57) **Abstract**

A humidifier (1) with safety elements, comprising a containment chamber (2) provided with at least one inlet hole (5) and at least one outlet hole (6) for an air stream and at least one delivery nozzle (3) for introducing liquid. The humidifier (1) comprises means for guiding the vertical translational motion of at least one first floating element (7a) and at least one second floating element (7b), which can move in a mutually independent manner on the liquid and have respective useful portions which are adapted to close the delivery nozzle (3) when a preset critical level is reached by the liquid.

## Description

The present invention relates to a humidifier with safety elements.

It is known that if a patient requires respiratory support by means of ventilators, the patient is supplied with adequately humidified air in order to allow simpler inhaling thereof.

For this purpose, it is known to use humidifiers provided with a heat source capable of heating the metal base of a humidification chamber, so as to evaporate the water introduced therein; the resulting water vapor mixes with the air, which can thus be sent to the patient.

Typically, the water introduced in the chamber arrives from a pouch or bottle arranged for example one meter above the chamber; from there, by mere gravity, the water descends within a tube until it reaches such chamber.

A particularly critical aspect of these humidifiers is control of the level of the water that is present in the chamber. It is in fact necessary to avoid the risk of exceeding a set maximum level, beyond which there would be the risk of sending water in the liquid state to the patient, with consequent danger of drowning.

To prevent this possibility, humidifiers provided with a float, which is arranged inside the chamber and moves to close the water delivery hole once the liquid has reached a certain level, are commercially available.

The presence of a single float inside the chamber, however, does not offer adequate assurances to protect the patient: a malfunction of such float is not infrequent, for example due to deformation of the coupled elements, which causes incorrect blockage of the delivery hole, or due to sticking of such float to the bottom of the chamber, which does not allow it to move to close the delivery hole.

In order to overcome this drawback, EP589429 B1 discloses a solution which resorts to a valve-type closure device provided with two floats.

Both floats are articulated to the walls of the humidification chamber and a respective element is articulated to each of them and, as a consequence of the rise of the level of the water, allows to prevent the introduction of water into the chamber.

In particular, the rise of the water level makes the first element, articulated to the first float, block the water delivery hole with its suitably shaped top.

If a malfunction occurs, the further increase in the level of the water moves the second element, which is articulated to the second float and is arranged coaxially with respect to the first float, so that it blocks a second hole, which connects the humidification chamber, connected to the patient, to a pre-chamber, along the walls of which the delivery hole is provided.

Therefore, in case of malfunction of the first float, the second float, which has less buoyancy than the first one, can activate in order to prevent the sending of water to the patient, allowing only the pre-chamber to fill.

Even this solution, however, is not free from drawbacks.

As is evident from the description given above, this is a solution of high complexity, due to the various articulations that are present and to the numerous elements that are needed to mutually connect the walls, the floats and the elements designed to close such holes.

Each float must in fact be connected to the walls of the chamber and to the element designed to close the water intake holes, by way of suitable guiding elements and hinges which are adapted to provide the desired articulations.

The presence of such a large number of elements therefore makes the production and/or assembly of the humidifier complicated; further, in order to ensure good operation of the device, it is necessary to resort to materials which have high-level mechanical characteristics, with a consequent cost increase.

It should be added to the above that the presence of such complex articulations in any case makes the humidifier significantly subject to failures, with consequent loss of reliability of the device, especially following frequent and repeated use.

It is also important to note that as a consequence of a malfunction of the first float the humidifier fills, albeit partially; the water can in fact enter through the delivery hole and fill the cavity formed by the pre-chamber. If the walls of such pre-chamber deteriorate, or otherwise have damage, even small damage, this leads to unwelcome outward dripping of water.

Finally, it should be noted that in the described solution the resistance to the air flow inside the chamber is particularly high, due to the large number of elements that are present and to their shape.

The aim of the present invention is to solve the above-mentioned drawbacks, by providing a humidifier which has great structural simplicity despite offering high assurances of safety against unintentionally sending water to the patient.

Within this aim, an object of the invention is to provide a humidifier which ensures high reliability in operation.

Another object of the invention is to provide a humidifier in which, as a consequence of the activation of any safety element, the input of water is prevented completely.

Another object of the invention is to provide a humidifier in which the resistance to the flow of air inside the chamber is low.

Another object of the invention is to provide a humidifier which can be obtained easily starting from commonly commercially available elements and materials.

Another object of the invention is to provide a humidifier which has low costs and is safe in application.

This aim and these and other objects, which will become better apparent hereinafter, are achieved by a humidifier with safety elements, which comprises a containment chamber provided with at least one intake hole and at least one outlet hole for an air stream and at least one delivery nozzle for introducing liquid, **characterized in that** it comprises means for guiding the vertical translational motion of at least one first floating element and at least one second floating element, which can move in a mutually independent manner on the liquid and have respective useful portions which are adapted to close said delivery nozzle when a preset level is reached by the liquid.

Further characteristics and advantages of the invention will become better apparent from the following detailed description of a preferred but not exclusive embodiment of the humidifier according to the invention, illustrated by way of non-limiting example in the accompanying drawings, wherein:
Figure 1 is an axial sectional view of the humidifier according to the invention with the closure element, according to a first possible embodiment, arranged in the open configuration;
Figures 2 and 3 are views, similar to Figure 1, of the humidifier with the closure element arranged in the closed configuration according to two different arrangements of the floating elements;
Figure 4 is a perspective view of the first floating element;
Figure 5 is a perspective view of the second floating element;
Figure 6 is a perspective view of the closure element of Figure 1;
Figure 7 is an axial sectional view of the closure element of Figure 1;
Figure 8 is a perspective view of the two floating elements and of the closure element in the mutual arrangement of Figure 1;
Figure 9 is an enlarged-scale axial sectional view of a detail of the humidifier and illustrates a different embodiment for the closure element.

With reference to the figures, a humidifier according to the invention, generally designated by the reference numeral 1, comprises a containment chamber 2, which has at least one delivery nozzle 3 for introducing liquid.

According to a solution of particular practical interest, the introduced liquid is water, and this liquid will be referenced in the continuation of the present description; in any case, embodiments which use a different type of liquid if required by the specific application also fall within the scope of the protection defined by this description.

The chamber 2 has at least one heated wall (for example the bottom 4, as in the embodiment shown in the accompanying figures) so as to cause evaporation of the water and allow the mixing of the resulting water vapor with a stream of air introduced in the chamber 2 by means of at least one inlet hole 5.

It is thus possible, by means of at least one outlet hole 6, to send the resulting humidified air stream for example to a patient who requires a respiratory assistance and for whom it is preferable to inhale air which has a high humidity content, as indeed the air produced by means of the humidifier 1.

According to the invention, the humidifier 1 comprises means for guiding the vertical translational motion of at least one first floating element 7a and of at least one second floating element 7b, both of which can move mutually independently on the water. The floating elements 7a, 7b have respective useful portions which are adapted to close the delivery nozzle 3 if the water, inside the chamber 2, reaches a preset level.

The two floating elements 7a, 7b thus act as safety elements, in the manners described hereinafter, to prevent the introduction of water in the chamber 2 (and therefore its sending to the patient) once the level of water inside the chamber 2 has reached this critical level.

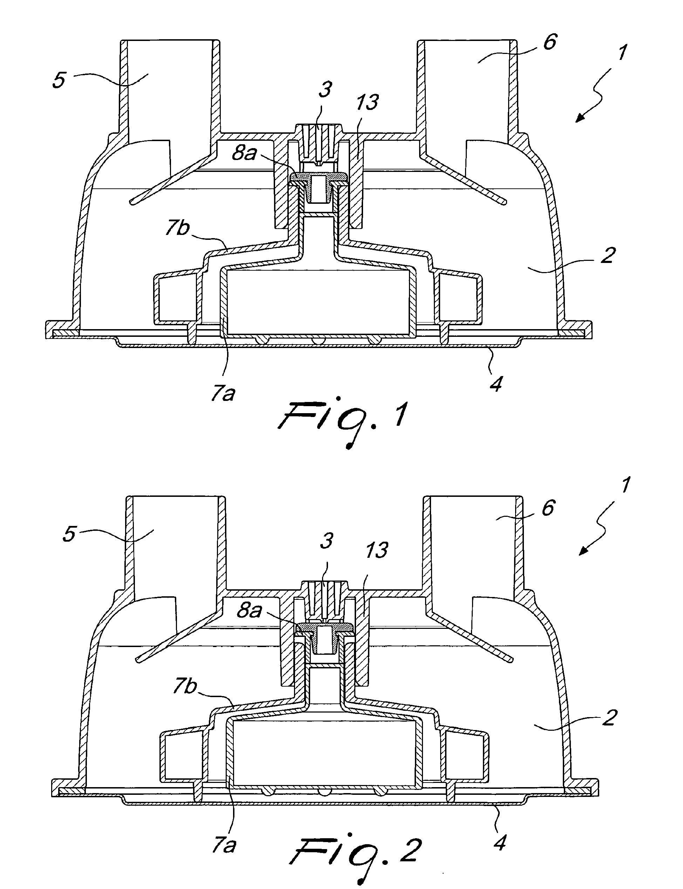

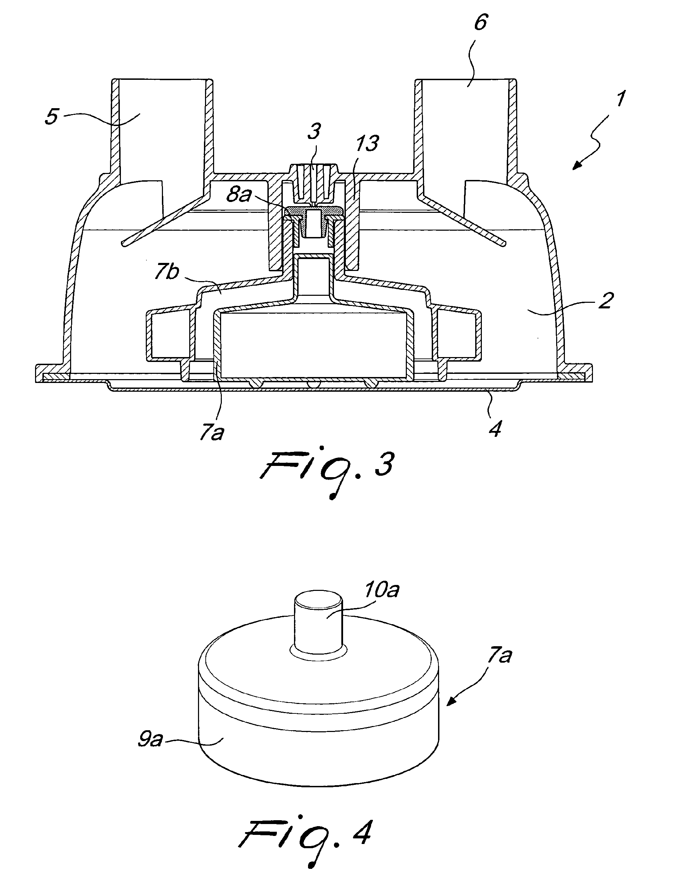

In particular, the second floating element 7b has less buoyancy than the first floating element 7a: if the water inside the chamber 2 reaches respectively a first predefined level and a second predefined level which is higher than the first one, correspondingly the useful portions of the first floating element 7a and of the second floating element 7b provide, separately and independently, the closure of the delivery nozzle 3, as shown in Figures 2 and 3.

The choice to cause the closure of the nozzle 3 at two different predefined levels by means of two mutually independent floating elements 7a, 7b conveniently allows to provide the humidifier 1 with two different safety elements.

The second floating element 7b in fact is activated only if the first one malfunctions and therefore the level of the water increases further beyond the first predefined level.

Advantageously, the humidifier 1 comprises at least one closure element 8a, 8b for the delivery nozzle 3, which is interposed between the delivery nozzle 3 and such useful portions of the floating elements 7a, 7b: the useful portions abut against the closure element 8a, 8b and can move to draw it to close the delivery nozzle 3 when one of the two predefined levels is reached.

It should be noted now that the described solution refers to a humidifier 1 which is provided with only two floating elements 7a, 7b, but the possibility to provide a larger number thereof, each having a different buoyancy and a respective useful portion for closing the nozzle 3 (or even a plurality of nozzles 3), is not excluded.

According to an embodiment of particular practical interest, which however does not limit the application of the invention, both the first floating element 7a and the second floating element 7b have a substantially axially symmetrical shape.

The two floating elements 7a, 7b comprise a wider base 9a, 9b, which floats on water, and a top 10a, 10b, which has a smaller diameter, abuts against the closure element 8a, 8b and constitutes such useful portions.

Conveniently, the top 10b of the second floating element 7b is connected to the respective base 9b, which has an annular shape, by means of a plurality of spokes 11, which are arranged radially around the axis of the second floating element 7b.

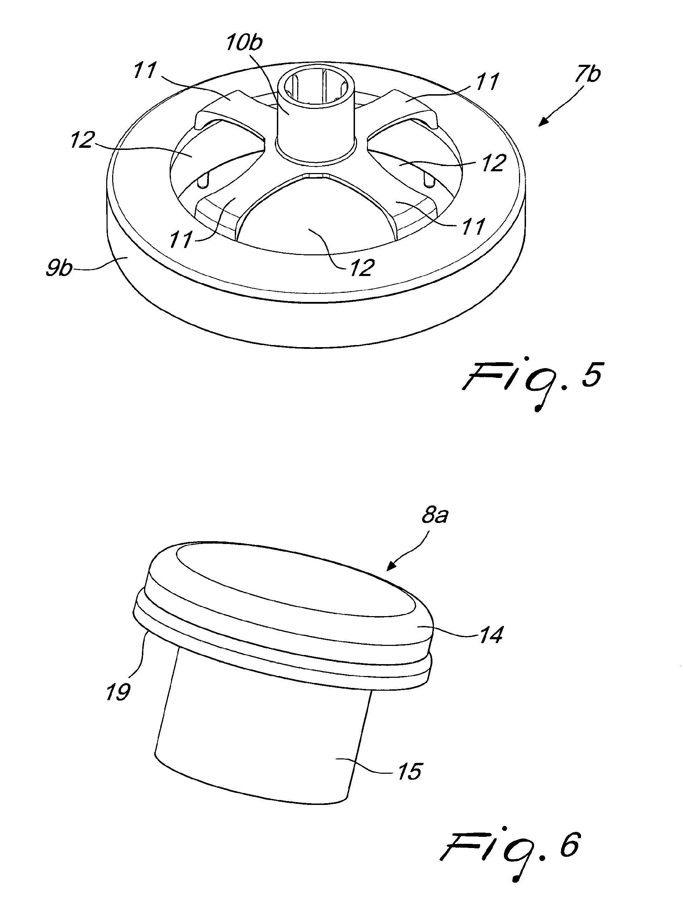

As shown particularly clearly in Figure 5, which shows a second floating element 7b provided with four angularly equidistant spokes 11, between each spoke 11 and the adjacent one there is an interspace 12, so as to allow the passage of air through the second floating element 7b.

In this manner, resistance to the air flow inside the chamber 2 is reduced considerably, particularly with respect to the devices of the background art, and it is thus possible to obtain internal turbulence adapted to facilitate the evaporation of water and the attainment of a high humidity content in the stream to be sent to the patient.

As can be deduced from the figures, both the base 9b and the top 10b of the second floating element 7b are hollow and the first floating element 7a is arranged coaxially inside the second one.

More particularly, the outer (cylindrical) lateral surface of the top 10a of the first floating element 7a can slide axially along the internal surface of the top 10b, which is tubular, of the second floating element 7b.

Such sliding occurs as a consequence of the progressive rise of the level of the water inside the chamber 2 (until the delivery nozzle 3 is closed by means of the first floating element 7a), due to the already noted mutually different buoyancy of the two floating elements 7a, 7b.

To ensure the provision of the closed configuration, as already noted, the humidifier 1 according to the invention comprises means for guiding the floating elements 7a, 7b.

More particularly, the guiding means comprise a sleeve 13, which is fixed internally to the upper wall of the chamber 2 and lies coaxially with respect to the delivery nozzle 3.

The top 10b of the second floating element 7b can slide within the sleeve 13: in this manner, while the guiding of the sliding of the first floating element 7a is ensured by the second floating element 7b, the latter is guided by the sleeve 13.

In summary, the correct mating between the delivery nozzle 3 and the closure element 8a, 8b, which is substantially contained in the sleeve 13 and is guided by it to move to close the nozzle 3, is thus ensured when one of the two predefined levels is reached by the water.

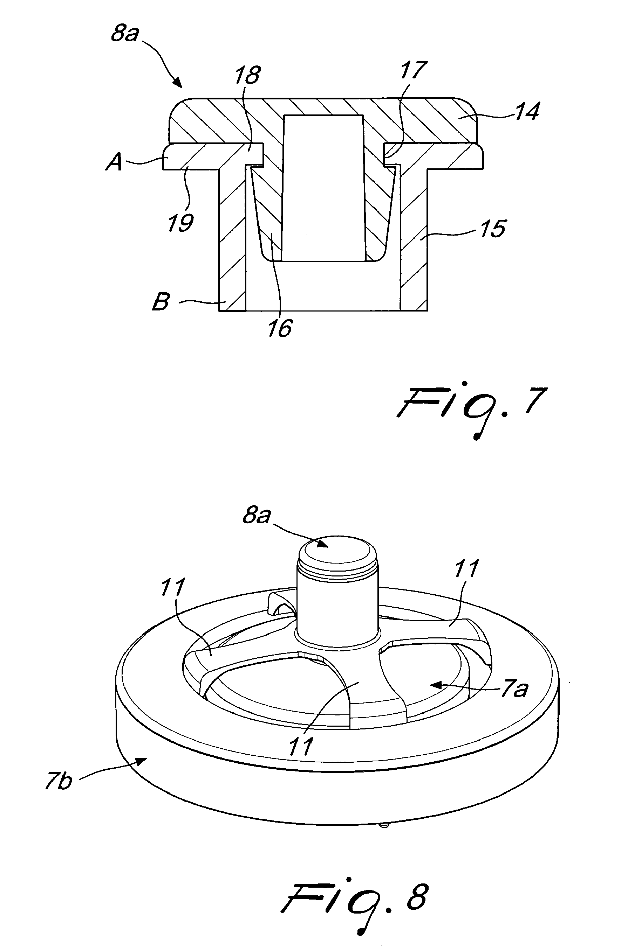

According to an embodiment of important practical interest, and with particular reference to Figures 6 and 7, the closure element 8a, 8b is shaped substantially like a plug and comprises a disk 14, which can engage the delivery nozzle 3 to close it, and a cylindrical support 15, which is fixed coaxially to the disk 14.

The fixing of the cylindrical support 15 to the disk 14 is provided by means of a tab 16, which protrudes coaxially below the disk 14 and has a notch 17, which allows a shape mating with a corresponding protrusion 18 formed inside the cylindrical support 15, which is hollow.

The cylindrical support 15 can slide along the internal surface of the top 10b of the second floating element 7b and has a shoulder 19, substantially at its upper end A, which is fixed to the disk 14.

The first floating element 7a abuts, with its top 10a, against the plug 8a at the lower end B of the cylindrical support 15 and slides with the plug 8a along the internal surface of the top 10b to move it to close the delivery nozzle 3 in the configuration shown in Figure 2.

If a malfunction of the first floating element 7a occurs, it is the second floating element 7b that acts on the plug 8a by way of its top 10b, which abuts against the shoulder 19, until the desired closure is provided in the configuration shown in Figure 3.

According to an alternative embodiment, the closure element 8a, 8b is constituted by a flexible membrane, which is rigidly coupled in a configuration which faces the delivery nozzle 3 and is proximate thereto. The tops 10a, 10b of the floating elements 7a, 7b are functionally associated with respective regions of the membrane 8b to provide, separately and independently, the closure of the delivery nozzle 3.

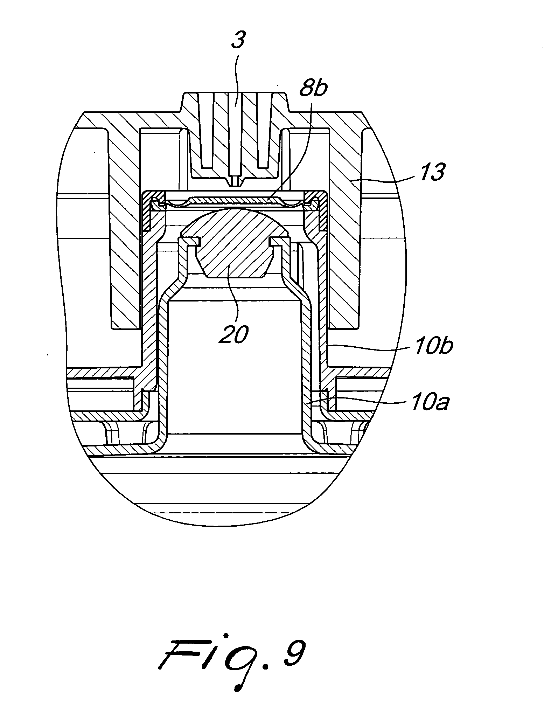

For example, as shown in Figure 9, the flexible membrane 8b has a disk-like shape and is fixed upward, at a peripheral region thereof, to the top 10b of the second floating element 7b.

In a manner similar to what has been described for the plug 8a which comprises the disk 14, the rise of the second floating element 7b on the water allows to move upward the membrane 8b, until the delivery nozzle 3 is closed, if the water reaches the second predefined level.

Also similarly to the previous described embodiment, the membrane 8b is moved to close by the second floating element 7b only as a consequence of a malfunction of the first floating element 7a.

The first floating element 7a is in fact provided with a head 20 which abuts against the central region of the membrane 8b which is disk-shaped and is capable of deforming it elastically to move it to close the delivery nozzle 3 if the water reaches the first predefined level.

The operation of the humidifier 1 according to the invention is as follows.

Due to a progressive rise of the level of the water inside the chamber 2, the two floating elements 7a, 7b arranged on the water rise, being guided by the sleeve 13, and move toward the delivery nozzle 3.

Particularly, due to the greater buoyancy, the superior buoyancy force that acts on the first floating element 7a causes it, when the water reaches the first predefined level, to draw the closure element 8a, 8b until the delivery nozzle 3 is closed.

If the first floating element 7a undergoes a malfunction, for example due to sticking of the latter to the bottom 4 of the chamber 2, the closure of the delivery nozzle 3 is ensured by the second floating element 7b. The second floating element 7b, too, rises (sliding inside the sleeve 13) as a consequence of the rise of the water beyond the first predefined level, and when the water reaches the second predefined level it draws the closure element 8a, 8b to close the delivery nozzle 3.

The second predefined level is therefore the maximum accepted limit for the level of the water, beyond which there would be the danger of sending water to the patient, with consequent risk of drowning.

It is evident that the described solution allows a particularly simple embodiment: the two floating elements 7a, 7b are free on the water and the sleeve 13 simply ensures their guiding to provide the closure configuration if the water reaches one of the two predefined levels.

The lack of complicated transmission devices and articulations, and the consequent structural simplicity, in addition to facilitating the operations for manufacturing and assembling the humidifier 1 according to the invention, is an assurance of reliability and reduces the risks of breakage which are otherwise possible due to frequent and repeated use.

Further, the structural simplicity does not force manufacturers to seek materials with high mechanical performance and therefore allows an advantageous cost saving.

Finally, since the closure element 8a, 8b, pushed by one of the two floating elements 7a, 7b, acts directly on the delivery nozzle 3, regardless of which floating element 7a, 7b is activated, the introduction of water into the chamber 2 is prevented completely. Therefore, differently from what instead occurred in the devices of the background art, there is no filling of a pre-chamber as a consequence of a malfunction of the first floating element 7a and therefore there is no risk of outward dripping in case of deterioration of the walls of the chamber 2.

In practice it has been found that the humidifier according to the invention fully achieves the proposed aim, since the presence of two floating elements which can move independently offers the necessary assurances of safety; at the same time, structural simplicity is ensured by resorting to simple means for guiding vertical translational motion to achieve closure of the delivery nozzle.

The invention thus conceived is susceptible of numerous modifications and variations, all of which are within the scope of the appended claims; all the details may further be replaced with other technically equivalent elements.

In the exemplary embodiments shown, individual characteristics, given in relation to specific examples, may actually be interchanged with other different characteristics that exist in other exemplary embodiments.

Moreover, it is noted that anything found to be already known during the patenting process is understood not to be claimed and to be the subject of a disclaimer.

In practice, the materials used, as well as the dimensions, may be any according to requirements and to the state of the art.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A humidifier with safety elements, comprising a containment chamber (2) provided with at least one inlet hole (5) and at least one outlet hole (6) for an air stream and at least one delivery nozzle (3) for introducing liquid, **characterized in that** it comprises means for guiding the vertical translational motion of at least one first floating element (7a) and at least one second floating element (7b), which can move in a mutually independent manner on the liquid and have respective useful portions which are adapted to close said delivery nozzle (3) when a preset level is reached by the liquid.

2. The humidifier according to claim 1, **characterized in that** said at least one second floating element (7b) has less buoyancy than said at least one first floating element (7a), said useful portions of said floating elements (7a, 7b) providing separately and independently the closure of said delivery nozzle (3) when the liquid reaches, inside said chamber (2), respectively a first predefined level and a second predefined level which is higher than the first one.

3. The humidifier according to claims 1 and 2, **characterized in that** it comprises at least one closure element (8a,8b) for said delivery nozzle (3), which is interposed between said useful portions of said floating elements (7a, 7b) and said nozzle (3), said useful portions abutting against said closure element (8a, 8b) and being movable to draw it to close said nozzle (3) when the liquid reaches said corresponding predefined levels.

4. The humidifier according to one or more of the preceding claims, **characterized in that** said first floating element (7a) and said second floating element (7b) have a substantially axially symmetrical shape and comprise a wider base (9a, 9b) and a top (10a, 10b) which has a reduced diameter and abuts against said closure element (8a, 8b), said top (10a, 10b) constituting said useful portion of said floating elements (7a, 7b).

5. The humidifier according to one or more of the preceding claims, **characterized in that** said top (10b) of said second floating element (7b) is connected to the respective said base (9b), which has an annular shape, by means of a plurality of spokes (11), which are arranged radially around the axis of said second floating element (7b), between each of said spokes (11) and the adjacent one there being at least one interspace (12) for the passage of air through said second floating element (7b).

6. The humidifier according to one or more of the preceding claims, **characterized in that** said base (9b) and said top (10b) of said second floating element (7b) are hollow, said first floating element (7a) being arranged coaxially within said second floating element (7b).

7. The humidifier according to claim 6, **characterized in that** the outer lateral surface of said top (10a) of said first floating element (7a) can slide coaxially along the internal surface of said top (10b), which has a tubular shape, of said second floating element (7b).

8. The humidifier according to one or more of the preceding claims, **characterized in that** said guiding means comprise a sleeve (13), which is fixed internally to the upper wall of said chamber (2) and runs coaxially with respect to said delivery nozzle (3), said top (10b) of said second floating element (7b) being able to slide within said sleeve (13).

9. The humidifier according to one or more of the preceding claims, **characterized in that** said closure element (8a, 8b) is shaped substantially like a plug (8a) and comprises a disk (14) which can engage said delivery nozzle (3) to close it and a cylindrical support (15) which is fixed coaxially to said disk (14).

10. The humidifier according to claim 9, **characterized in that** said cylindrical support (15) can slide along the internal surface of said top (10b) of said second floating element (7b) and has a shoulder (19) substantially at its upper end (A), which is fixed to said disk (14), said top (10b) of said second floating element (7b) abutting against said shoulder (19), said top (10a) of said first floating element (7a) abutting against the lower end (B) of said cylindrical support (15).

11. The device according to one or more of claims 1 to 8, **characterized in that** said closure element (8a, 8b) is constituted by a flexible membrane (8b), which is coupled in a configuration which faces said delivery nozzle (3) and is proximate thereto, said tops (10a, 10b) of said floating elements (7a, 7b) being functionally associated with respective regions of said membrane (8b) to provide separately and independently the closure of said delivery nozzle (3).
